# EUROPÄISCHE PATENTSCHRIFT

(11) **EP 1 987 848 B1**
(45) Veröffentlichungstag und Bekanntmachung des Hinweises auf die Patenterteilung: **10.07.2013**
(21) Anmeldenummer: 08008425.4
(22) Anmeldetag: 05.05.2008
(51) Int. Cl.: A61L 9/012, E03D 9/00

(54) **Schwimmende Sperrschicht**
Floating barrier layer
Couche de blocage flottante

(30) Priorität: 04.05.2007 DE 202007006533 U
(43) Veröffentlichungstag der Anmeldung: 05.11.2008
(73) Patentinhaber: Miorin, Martin, 87437 Kempten (DE); Reisacher, Hannes, 87471 Weidach (DE)
(72) Erfinder: Miorin, Martin, 87437 Kempten (DE); Reisacher, Hannes, 87471 Weidach (DE)
(74) Vertreter: Fiener, Josef

(56) Entgegenhaltungen:
- WO-A-01/42171
- WO-A-02/095148
- WO-A-03/056900
- DD-A- 300 453
- DE-A1- 19 714 869
- DE-B- 1 261 806
- DE-U1- 29 622 384
- US-A- 4 278 206

## Beschreibung

Die Erfindung betrifft eine schwimmende Sperrschicht mit den oberbegrifflichen Merkmalen des Anspruchs 1. Eine solche Sperrschicht aus Granulaten, Blähton, Perlit, Kieselsäure od. dgl. wird in der WO 03/056900 beschrieben, jedoch für großflächige Klär oder Güllebecken in der Landwirtschaft.

Die Privathaushalte verbrauchten im Jahre 2005 in Deutschland durchschnittlich 126 I Trinkwasser pro Tag, wovon allein 40 I für den Betrieb von WC-Spülungen verwendet wurden. Es entsteht hierdurch eine beachtliche Menge an Abwässern, die über die Kanalisation abtransportiert und zur Aufreinigung in Kläranlagen gesammelt und aufwändig behandelt werden müssen, bevor die Abwässer wieder dem Wasserkreislauf zugeführt werden können.
Der Wasserverbrauch ist zwar seit den 1990er Jahren deutlich gesunken, es bestehen dennoch v.a. im WC-Bereich ungenutzte Potentiale zur Wassereinsparung. Einen Ansatz hierfür bieten beispielsweise technische Verbesserungen an WC-Spüleinrichtungen und wassersparenden Armaturen. Der höchste Spareffekt wird jedoch erzielt, wenn die Toilettenspülung nicht nach jeder Benutzung erfolgt. Nachteilig daran ist allerdings, dass dabei entweder durch den Eigengeruch der abgesetzten Exkremente oder aufgrund des bakteriellen Abbaus eine erhebliche Geruchsbelästigung in den WC-Räumen entsteht. Ein länger andauernder Spülverzicht ist somit ohne den zusätzlichen Einsatz wenig umweltfreundlicher, geruchsbindender Chemikalien oder starker synthetischer Duftstoffe nicht zumutbar.

Zur Abmilderung dieses Problems schlägt die DE 20 23 133 ein Verfahren zum geruchlosen Ablegen von Exkrementen in einem mit einer Wasserspülung üblicher Art versehenen Klosettbecken vor, bei dem vor dem Ablegen der Exkremente mit einem in Tablettenform oder in einer Aerosoldose vorliegenden schaumbildenden Präparat in der Klosettschüssel ein Schaumkissen erzeugt wird und beim Betätigen der Wasserspülung zusammen mit den Exkrementen entfernt wird.

Ebenfalls eine Schaumabdeckung der Klosettschüssel zeigt die WO 87/06289, wobei der Schaumteppich hier aus einen Mehrkomponentensystem besteht und erst bei Mischung der Komponenten in der Klosettschüssel gebildet wird. Vor dem Spülen wird der Schaum jedoch mittels eines den Schaum chemisch abbindenden Mittels aufgelöst, und anschließend ein neuer Schaumteppich erzeugt. Der Schaumteppich bleibt dann über einen Zeitraum von 10 bis 40 Minuten stabil.

Die WO 02/095148 zeigt wasserlösliche Beutel mit einer biologisch abbaubaren Zusammensetzung, die nach Einbringen in eine WC-Schüssel und Auflösen des Beutels antimikrobiell und dadurch geruchsmindernd wirkt.

Dabei besteht die Gefahr, dass der Beutel oder die Folie bei der WO 03/056 900 absinkt und verklumpt.

Nachteilig an den genannten Systemen ist, dass im erstgenannten Fall die Schaumschicht relativ aufwändig mittels Tabletten oder einer Aerosoldose erzeugt werden muss, und nicht die Einsparung von Wasser, sondern die Geruchsbindung im Vordergrund steht. Diese Aufgabe wird durch Zugabe bzw. den Eintrag schaumbildender Chemikalien ohne Rücksicht auf die damit verbundene Umweltbelastung gelöst. Die erzeugten Schäume sind zudem in beiden Fällen nur für relativ kurze Zeit stabil und müssen vor jeder Toilettenbenutzung neu gebildet werden. Die in Beuteln bevorratete Zusammensetzung trägt zwar zur Wassereinsparung bei, wird dazu aber in das Toilettenwasser eingebracht und kann erst nach Auflösen der Beutel die Geruchsbildung chemisch unterbinden.

Aufgabe der vorliegenden Erfindung ist es daher, eine Sperrschicht zur Verfügung zu stellen, durch die auch ohne Spülvorgang eine Geruchsbildung durch Urin wirkungsvoll unterbunden wird und die dabei kostengünstig und biologisch unbedenklich ist, sich einfach erzeugen lässt und über einen längeren Zeitraum in einem Urinal stabil bleibt.

Die Aufgabe wird gelöst durch eine schwimmende Sperrschicht gemäß Anspruch 1. Vorteilhafte Weiterbildungen der Erfindung sind Gegenstand der abhängigen Ansprüche.

Die erfindungsgemäße Sperrschicht ist aus mehreren Bestandteilen zusammengesetzt, wobei 25 - 99 Gew.-%, insbesondere 50 - 75 Gew.-% aus anorganischen Metalloxiden bestehen. Der durch die anorganischen Metalloxide gebildete Anteil besteht dabei insbesondere aus Kieselsäure (SiO₂), wobei gefällte Kieselsäure oder pyrogene Kieselsäure sowie Mischungen daraus Verwendung finden. Darüber hinaus können selbstverständlich alle weiteren geeigneten Metalloxide, wie beispielsweise Aluminiumoxide oder aber Mischoxide verwendet werden. Auch kann die als Nebenprodukt bei der Herstellung pyrogener Kieselsäure anfallende Filterfraktion als Haupt - oder Teilbestandteil der erfindungsgemäßen Sperrschicht eingesetzt werden. Durch die Verwendung von Metalloxiden, besonders von SiO₂, in Form von pyrogener oder gefällter Kieselsäure, kann eine besonders kostengünstige Sperrschicht erzeugt werden. Seitens des Anwender- wie auch des Umweltschutzes bestehen keine Bedenken gegenüber der Verwendung von Kieselsäure. Diese ist hypoallergen und bei oraler oder dermaler Aufnahme treten keine akut toxischen Symptome auf. Kieselsäure ist zudem ökologisch weitgehend unbedenklich und hat keinen Einfluss auf das Grundwasser. Die gefällte oder pyrogene Kieselsäure ist ein anorganischer, schwer wasserlöslicher Stoff und ihre Bioverfügbarkeit für aquatische Organismen daher gering. Daraus ergibt sich, dass chronische Effekte und eine Anreicherung in Organismen nicht auftreten. Die erfindungsgemäße Sperrschicht stellt somit eine wesentlich kostengünstigere und dabei umwelt- oder gesundheitsfreundliche Alternative zu Schaumschichten oder chemischen Geruchssperren oder Geruchsabsorbern dar.

Da eine schwimmende, geschlossene Sperrschicht ausgebildet werden soll, weist zumindest ein Teil der Sperrschicht hydrophobe Eigenschaften auf. Diese Hydrophobizität wird erreicht, indem die Zusammensetzung der Sperrschicht 1 - 50 Gew.-% insbesondere aber 1 - 25 Gew.-% wenigstens eines Hydrophobierungsmittels enthält. Die anorganischen Metalloxide werden in der Regel durch chemische Modifikation ihrer freien OH-Gruppen hydrophobiert. Die Hydrophobierung erfolgt beispielsweise nach Fällung oder Pyrolyse der Kieselsäure und weitgehender Umsetzung freier OH-Gruppen der Kieselsäure mit z.B. Silanen, Silazanen oder Siloxanen. Dabei sind Hexadecylsilan, Dimethyldichlorsilan, Hexamethyldisilazan, Octamethylcyclotetrasiloxan, Polydimethylsiloxan oder Methacrylsilane bevorzugte Hydrophobierungsmittel.

Geeignete Hydrophobierungsmittel können als eigenständiger Bestandteil zum jeweils verwendeten anorganischen Metalloxid beigemischt werden oder aber direkt nach der Synthese (Fällung oder Pyrolyse) zu dem Anteil der Sperrschicht zugesetzt werden, der aus anorganischen Metalloxiden gebildet wird. Optional enthält die Sperrschicht des Weiteren wenigstens einen Zusatz- oder Füllstoff, der die Sperrschicht weiter funktionalisiert. Aufgrund ihrer hydrophoben Eigenschaften bleibt die Sperrschicht über mehrere Spülvorgänge oder bei längeren Standzeiten bei wenig benutzten Toiletten oder Abflüssen stabil. Erst der längere Kontakt mit Flüssigkeit senkt die Hydrophobizität der Zusammensetzung. Je nach Art der verwendeten Zusatzstoffe bleiben deren beispielsweise antimikrobiellen Bestandteile auch nach dem teilweisen Absinken weiter aktiv.

Das Sperrschicht-Materialist in Art eines "Pads" auf einem Trägerstoff, aufgebracht, wobei das Sperrschichtmaterial auf dem Wasser gleichmäßig aufschwimmt. Diese Form kann somit in Art des bekannten Toilettenpapiers als Rollenware eingesetzt werden. Die Sperrschicht eignet sich zur Verwendung in Tief- und Flachspültoiletten sowie in Urinalen und Pissoirs, darüber hinaus aber auch zur Geruchsabsperrung von Abflüssen bzw. Siphons oder als umweltfreundliche Alternative oder Ergänzung zu den üblicherweise in mobilen Toilettenkabinen und Sanitärcontainern verwendeten Chemikalien.

Vorteilhafterweise kann die Sperrschicht auch aus Metalloxid gebildet werden, das ganz oder teilweise hydrophob ist. Hierfür wird u. U. auf ein herstellerseitig vorbehandeltes Ausgangsmaterial zurückgegriffen. Der prozentuale Anteil an zusätzlich zur Sperrschicht zugesetztem Hydrophobierungsmittel wird dann entsprechend reduziert.

Als besonders vorteilhaft wird angesehen, wenn die Bestandteile der Sperrschicht ganz oder teilweise granuliert sind. Im Zuge der Granulierung, die beispielsweise in einer speziellen Granuliermühle erfolgen kann, können die Bestandteile der Sperrschicht weiter modifiziert werden. Bevorzugt werden dabei die anorganischen Metalloxide in Anwesenheit von Hydrophobierungsmitteln granuliert und dabei durch diese modifiziert oder mit diesen verbunden. Daraus ergeben sich weitere Vorteile, da auf ein kostengünstigeres Ausgangsmaterial zurückgegriffen werden kann, das dennoch nach der Granulierung den gewünschten Hydrophobizitätsgrad aufweist, um die erfindungsgemäße schwimmende Sperrschicht mit guten Standzeiten zu bilden. Durch die Granulierung wird auch die Neigung zur Staubbildung, die das metalloxidische Ausgangsmaterial in der Regel aufweist, wirksam reduziert bzw. vollständig unterbunden. Im Verlauf des Granulierungsprozesses kann zudem die Partikelgröße der Granulatbestandteile eingestellt und damit auf die Eigenschaften des verwendeten Ausgangssubstrates abgestimmt werden, um letztendlich eine homogene und gut schwimmfähige Sperrschicht zu erhalten. Die Granulierung kann aber auch ohne die Anwesenheit von Hydrophobierungsmitteln erfolgen. Diese werden in einer alternativen Ausführungsform der Erfindung dann noch nachträglich zugesetzt.

Eine weitere vorteilhafte Ausführungsform der Erfindung sieht vor, dass das anorganische Metalloxid eine hydrophobe Oberflächenbeschichtung aufweist. Hierbei entfällt eine chemische Umsetzung der freien OH-Gruppen der Kieselsäure, da die Oberflächenbeschichtung bevorzugt als "coating" aus Wachs oder anderen hydrophobierenden Polymeren, beispielsweise auf Polyurethanbasis o. dgl. gebildet wird.

Die Sperrschicht weist zusätzlich einen variablen Anteil an Zusatzstoffen auf. Je nach Verwendungszweck handelt es sich hierbei bevorzugt um Biozide, Desodorantien, Rieselhilfen, Konservierungsmittel und/oder Farbstoffe. Durch den Zusatz von antibiotisch wirkenden Substanzen kann die Keimzahl im WC gesenkt werden. Daraus wird zum einen dem Hygienebedürfnis des Toilettenbenutzers entsprochen, zum anderen kann die Zahl der Bakterien, die für die Bildung unangenehmer Gerüche mitverantwortlich sind, reduziert werden. Soll die Sperrschicht beispielsweise auf Autobahnrastplätzen oder Bahnhofstoiletten, über den Zeitraum von mehreren Tagen bis Wochen in den WC-Becken oder Urinalen verbleiben, können auch Algizide oder Fungizide zugesetzt werden. Um trotz der Sperrschicht entweichende Gerüche wirkungsvoll zu kaschieren, können als Zusatzstoff Desodorantien beigemischt werden, die bei kontinuierlicher Abgabe einen angenehmen Geruch vermitteln. Als Desodorans eignet sich beispielsweise Chloramin T, das als Oxidationsmittel auch eine hohe bakterizide Wirksamkeit aufweist und zusätzlich als Desinfektionsmittel und Antiseptikum wirkt. Da die Sperrschicht aus Bestandteilen gebildet wird, die bevorzugt granuliert vorliegen, kann u. U. das Verklumpen der Bestandteile auftreten. Um dies zu verhindern werden hygroskope Zusatzstoffe wie beispielsweise Kreide oder Magnesiumkarbonat in geringen Mengen beigemischt.

Als weitere Zusatzstoffe können der Zusammensetzung Farbstoffe hinzugefügt werden, um die Sperrschicht optisch aufzuwerten. So werden beispielsweise die Farben blau und gelb, im Allgemeinen als besonders frisch empfunden und eine entsprechend gefärbte Sperrschicht besser von den Benutzern akzeptiert. Zusätzlich zu den bereits genannten Zusatz- und Hilfsstoffen können als weiterer Bestandteil Zeolithe beigegeben werden. Aufgrund der Struktur können diese Silikat-Minerale verschiedene Stoffe einlagern, im Kontakt mit Wasser über einen längeren Zeitraum wieder freisetzen und dabei die Sperrschicht funktionalisieren. In synthetische Zeolithe, wie beispielsweise in Zeolith A werden so durch Ionentausch Silberionen eingelagert, wodurch ein Biozid-Depot mit langdauernder antimikrobieller Wirkung geschaffen wird.

Der Sperrschicht können ein einziger oder aber auch mehrere Zusatzstoffe zugesetzt werden, wobei es als besonders vorteilhaft angesehen wird, wenn der oder die Zusatzstoff(e) durch Granulierung mit dem anorganischen Metalloxid verbunden ist (sind). Dadurch wird sichergestellt, dass das Verhältnis der Zusammensetzung nur in minimalen Grenzen schwankt und während Lagerung und/oder Transport keine Entmischung der Zusammensetzung stattfindet.

Daneben bringt es auch Vorteile mit sich, wenn der oder die Zusatzstoff(e) nachträglich, also nach der Granulierung zugesetzt werden. Somit lässt sich die Zusammensetzung besser auf verschiedene Einsatzort der Sperrschicht und auf unterschiedliche Verbraucherwünsche abstimmen. Wird die Sperrschicht beispielsweise in öffentlichen WCs verwendet und steht hier der antimikrobielle Wirkaspekt im Vordergrund, kann eine Grundzusammensetzung nachträglich mit bioziden Zusatzstoffen versetzt werden, um hier die gewünschte Wirkung zu erzielen. Bei der Verwendung im Privathaushalt wird u. U. ein angenehmerer Geruch und eine ansprechendere Farbgebung höher bewertet, weshalb hier verstärkt entsprechende Farb- und Geruchsstoffe zugefügt werden.

Weitere Vorteile, Merkmale und Besonderheiten der Erfindung ergeben sich aus der nachfolgenden Beschreibung einer bevorzugten, jedoch nicht beschränkenden Ausführungsform der Erfindung anhand der schematischen Zeichnung. Es zeigt:

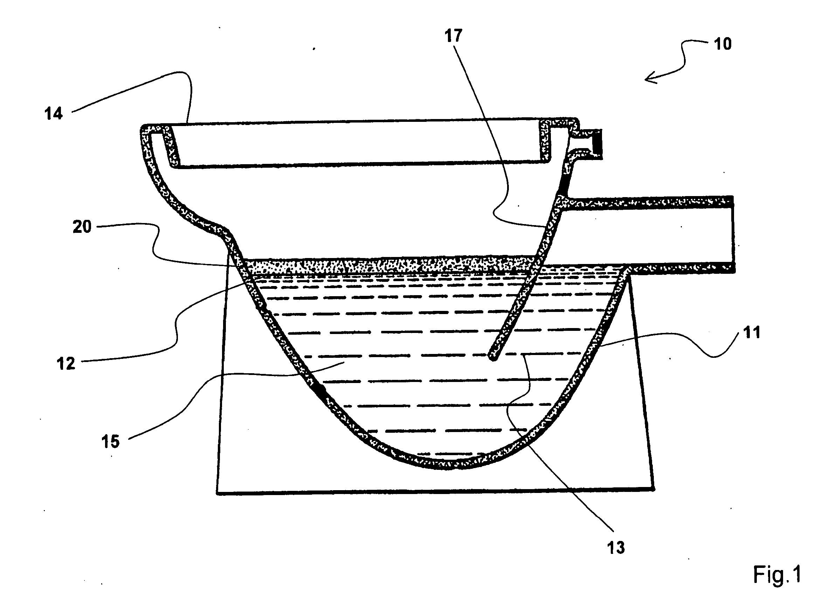

- Fig.1: eine bevorzugte Ausführungsform der erfindungsgemäßen Sperrschicht in einem WC-Becken.

Fig.1 zeigt das WC-Becken 11 einer Toilette 10. Die in Fig.1 dargestellte Toilette 10 funktioniert im Tiefspülprinzip, d.h. die Ausscheidungen fallen in das im WC-Becken 11 angestaute Toilettenwasser 15, das einen Siphon 13 füllt, der sich unter dem Beckenrand 14 befindet. Die erfindungsgemäße Sperrschicht 20 schwimmt auf der Wasseroberfläche 12 des Toilettenwassers 15. Die Ausscheidungen fallen in das WC-Becken 11, dringen dort durch die Sperrschicht 20 und werden dann vom darunter liegenden Toilettenwasser 15 aufgenommen. Bei der Abgabe von Urin wird ein geringer Teil von der Sperrschicht 20 absorbiert. Aufgrund der Hydrophobie der Bestandteile der Sperrschicht 20 durchdringt der wesentlich größere Anteil jedoch die Sperrschicht 20, wird ebenfalls vom Toilettenwasser 15 aufgenommen und durch dieses verdünnt. Trotz des Verdünnungseffektes würde ohne Spülvorgang sehr bald eine Geruchsemission aus dem WC-Becken 11 stattfinden. Aufgrund der Sperrschicht 20 kann diese Geruchsbelästigung allerdings über einen langen Zeitraum und über mehrere Toilettenbenutzungen hinweg unterbunden werden, ohne dass ein Spülvorgang notwendig wird. Aufgrund der zur Sperrschicht 20 beigemischten Duftstoffe, die zeitverzögert freigesetzt werden, herrscht immer ein angenehmes Raumklima, so dass auch nach längerer Zeit kein Anreiz besteht, die Toilettenspülung zu betätigen.

Weitere Verursacher von unangenehmen Gerüchen sind Urintröpfchen, die beim Auftreffen des Urinstrahls auf der Innenwand 17 des WC-Beckens 11 oder der Wasseroberfläche 12 in Toiletten 10 ohne Sperrschicht 20 spritzen. Diese Tröpfchen werden durch die Sperrschicht 20 weitestgehend aufgenommen und eine Geruchsbildung wirksam unterbunden. Da diese u. U. mit Keimen belasteten Spritzer durch die Sperrschicht 20 unterbunden werden, sind Toiletten 10 mit Sperrschicht 20 für den Benutzer wesentlich hygienischer.

Wird die Wasserspülung benutzt, bleibt die Sperrschicht 20 aufgrund ihrer hydrophoben Bestandteile über mehrere Spülvorgänge hinweg im WC-Becken 11 stabil. Erst mit zunehmender Durchdringung durch das Toilettenwasser 15 und wegen des sich eventuell allmählich ändernden pH-Wertes, nimmt die Hydrophobizität der die Sperrschicht 20 bildenden Bestandteile ab. Die Sperrschicht 20 eignet sich auch zur Verwendung in Flachspültoiletten, in Urinalen, Pissoirs, in Abflüssen oder als umweltfreundliche Alternative zu entsprechenden Chemikalien in mobilen Toilettenkabinen und Sanitärcontainern.

### Bezugszeichenliste:

10 = Toilette
11 = WC-Becken
12 = Wasseroberfläche
13 = Siphon
14 = Beckenrand
15 = Toilettenwasser
17 = Innenwand
20 = Sperrschicht

## Patentansprüche

1. Schwimmende Sperrschicht (20), die aus 25 - 99 Gew.-%, insbesondere 50 - 75 Gew.-% wenigstens eines anorganischen Metalloxids, insbesondere Kieselsäure zusammengesetzt ist, wobei 1 - 25 Gew.-% eines Hydrophobierungsmittels und optional wenigstens ein Zusatzstoff vorgesehen ist, **dadurch gekennzeichnet, dass**
das anorganische Metalloxid zwischen einem Trägerstoff in Art eines Pads angeordnet ist.

2. Sperrschicht (20) nach Anspruch 1, **dadurch gekennzeichnet, dass**
das anorganische Metalloxid eine hydrophobe Oberflächenbeschichtung aufweist.

3. Sperrschicht (20) nach Anspruch 2, **dadurch gekennzeichnet, dass**
die Oberflächenbeschichtung aus Wachs oder Polymer gebildet ist.

4. Sperrschicht (20) nach Anspruch 1, **dadurch gekennzeichnet, dass**
das Hydrophobierungsmittel durch Granulierung mit den anorganischen Metalloxiden verbunden ist.

5. Sperrschicht (20) nach einem der Ansprüche 1 - 4, **dadurch gekennzeichnet, dass**
der Zusatzstoff Biozide, Desodorantien, Rieselhilfen, Konservierungsmittel und/oder Farbstoffe umfasst.

6. Sperrschicht (20) nach einem der Ansprüche 1 - 5, **dadurch gekennzeichnet, dass**
der Zusatzstoff durch Granulierung mit dem anorganischen Metalloxid verbunden ist.

7. Sperrschicht (20) nach einem der Ansprüche 1 - 5, **dadurch gekennzeichnet, dass**
der Zusatzstoff nach der Granulierung zugesetzt ist.

8. Sperrscicht (20) nach einem der Ansprüche 1 - 7, **dadurch gekennzeichnet, dass**
das Pad geeignet zum Verbleib über mehrere Tage in einem Urinal ist.

## Claims

1. Floating barrier layer (20), composed of 25 - 99 wt-%, particularly 50 - 75 wt-% of at least one inorganic metal oxide, in particular silica, wherein 1 - 25 wt-% of a hydrophobizing agent, and optionally at least one additive are provided, **characterized in that**
the inorganic metal oxide is arranged between a carrier material in form of a pad.

2. Barrier layer (20) of claim 1, **characterized in that**
the inorganic metal oxide has a hydrophobic surface coating.

3. Barrier layer (20) according to claim 2, **characterized in that**
the surface coating is formed of wax or polymer.

4. Barrier layer (20) of claim 1, **characterized in that**
the hydrophobizing agent is linked with the inorganic metal oxides by granulation.

5. Barrier layer (20) of any of claims 1 to 4, **characterized in that**
the additive includes biocides, deodorants, anti-caking agents, preserving agents and/or dyes.

6. Barrier layer (20) of any of claims 1 to 5, **characterized in that**
the additive is linked with the inorganic metal oxide by granulation.

7. Barrier layer (20) of any of claims 1 to 5, **characterized in that**
the additive is added after the granulation.

8. Barrier layer (20) of any of claims 1 to 7, **characterized in that**
the pad is adapted to stay in a urinal for several days.

## Revendications

1. Couche de barrage flottante (20), qui est composée de 25 à 99 % en poids, en particulier de 60 à 75 % en poids, d'au moins un oxyde métallique inorganique, en particulier d'acide silicique, sachant qu'il est prévu 1 à 25 % en poids d'un agent hydrophobe et, optionnellement, au moins un additif, **caractérisée en ce que** l'oxyde métallique inorganique est disposé à la manière d'un tapis sur un matériau porteur.

2. Couche de barrage (20) selon la revendication 1, **caractérisée en ce que** l'oxyde métallique inorganique présente un revêtement de surface hydrophobe.

3. Couche de barrage (20) selon la revendication 2, **caractérisée en ce que** le revêtement de surface est formé d'une cire ou d'un polymère.

4. Couche de barrage (20) selon la revendication 1, **caractérisée en ce que** l'agent hydrophobe est lié par granulation aux oxydes métalliques inorganiques.

5. Couche de barrage (20) selon l'une des revendications 1 à 4, **caractérisée en ce que** l'additif comprend des biocides, des déodorants, des aides au ruissellement, des agents de conservation et/ou des colorants.

6. Couche de barrage (20) selon l'une des revendications 1 à 5, **caractérisée en ce que** l'additif est lié par granulation à l'oxyde métallique inorganique.

7. Couche de barrage (20) selon l'une des revendications 1 à 5, **caractérisée en ce que** l'additif est ajouté après la granulation.

8. Couche de barrage (20) selon l'une des revendications 1 à 7, **caractérisée en ce que** le tapis est apte à rester pendant plusieurs jours dans un urinoir.
